# EUROPEAN PATENT APPLICATION

(11) **EP 4 693 175 A1**
(43) Date of publication of application: **11.02.2026**
(21) Application number: 24192902.5
(22) Date of filing: 05.08.2024
(51) Int. Cl.: G06T 7/00, G06T 7/30, G06T 19/20, G16H 30/40, G16H 50/20

(54) **METHOD AND SYSTEM FOR EVALUATING 3D MODELS OF A DENTAL SITUATION**

(71) Applicant: 3Shape A/S, 1060 Copenhagen K (DK)
(72) Inventor: CEBOV, Aleksandar, 1060 Copenhagen K (DK); KADIEV, Kostadin, 1060 Copenhagen K (DK)
(74) Representative: Guardian IP Consulting I/S

(57) **Abstract**

A computer-implemented method for evaluating digital 3D models of teeth is disclosed. The method comprises receiving a first digital 3D model (100) representative of the dental situation at a first time and receiving a second digital 3D model (101) representative of the dental situation at a second time. Further, the method comprises generating a superimposed digital 3D model by mutually aligning the first digital 3D model (100) and the second digital 3D model (101) and displaying a part of the superimposed digital 3D model comprising a lesion (106) of a dental condition identified on the first digital 3D model (100) and on the second digital 3d model (101). The displaying comprises arranging a user-adjustable controller (200) to vertically divide the displayed part of the superimposed model into a first region (201) and a second region (202), wherein in the first region (201) only a part of the first digital 3D model (101) of the superimposed digital 3D model is rendered, and wherein in the second region (202) only a part of the second digital 3D model (101) of the superimposed digital 3D model is rendered. The displaying further comprises identifying a first end point (208) of the lesion (106) in the first region (201) and a second end point (209) of the lesion (106) in the second region (202), arranging a first interactive window (203) at any point of a display device with an x coordinate less than, or equal to the x coordinate of the first end point (208) of the lesion (106), the first interactive window (203) comprising a severity value of the lesion (106) identified on the first digital 3D model (100), and arranging a second interactive window (204) at any point of the display device with the x coordinate greater than, or equal to the x coordinate of the second end point (209) of the lesion (106), the second interactive window (204) comprising a severity value of the lesion (106) identified on the second digital 3D model (101).

## Description

### Technical field

The disclosure relates to a computer-implemented method and system for evaluating digital three-dimensional (3D) models of teeth. In particular, the disclosure relates to a computer-implemented method and system for arranging two-dimensional interactive windows with respect to superimposed digital three-dimensional (3D) models such that both a lesion of a dental condition and the interactive windows can be observed.

### Background

Development of intraoral scanning techniques has been instrumental in the transition to modern digital dentistry. Use of intraoral 3D scanners allows dental practitioners to accurately and quickly capture dental situation of a patient, which may then be visualized on a display as a digital three-dimensional (3D) model. Obtained digital 3D model may thus serve as a digital impression of teeth, offering numerous advantages over a classical physical impression of teeth.

Scanning of the dental situation of the same patient over a course of time may result in a series of digital 3D models which may be compared to each other for, for example, tracking changes in development of dental conditions such as plaque, caries, gingivitis, dental recession, malocclusion, dental cancer, tooth wear or other. Presenting identified lesions of the dental condition on a plurality of digital 3D models, as well as the annotations describing the lesions, may be challenging in the digital 3D environment. Known methods including side-by-side view, or a sequential view of digital 3D models do not offer sufficient flexibility for the user to inspect the change in the dental condition without relying heavily on their individual skill. Furthermore, when interacting with the digital 3D models, for example when rotating the digital 3D models, the user may lose from sight the annotations describing the identified lesions and/or the lesion itself as both the annotations and or the lesions may be hidden or misplaced during the interaction with the digital 3D models.

Another limitation of the current solutions for annotating parts of the digital 3D models is that they are limited with respect to the user interaction due to difficulty of implementing interactive annotations in the digital 3D space. Currently available annotations such as text boxes, drop-down menus, input handler events are mature solutions in the 2D space. It would be beneficial to develop similar solutions in the digital 3D space.

There is a need to develop methods and systems to enable continuous conveying of the clinically relevant annotations, about how the dental condition is changing, during displaying of the digital 3D models and during user interaction with the displayed digital 3D models. Furthermore, enabling user interaction with the annotations during displaying of the dental conditions on the digital 3D models is desirable.

The present disclosure addresses how to utilize the capabilities of the intraoral 3D scanner systems to allow the user to review the identified lesions of dental condition on a plurality of digital 3D models, to inspect and interact with the associated annotations and to influence the diagnostic capability of the intraoral 3D scanner system based on the interaction.

### Summary

In an embodiment, a computer-implemented method for evaluating digital 3D models of teeth is disclosed, the method comprising:
- receiving a first digital 3D model representative of the dental situation at a first time,
- receiving a second digital 3D model representative of the dental situation at a second time,
- generating a superimposed digital 3D model by mutually aligning the first digital 3D model and the second digital 3D model,
- displaying a part of the superimposed digital 3D model comprising a lesion of a dental condition identified on the first digital 3D model and on the second digital 3d model, wherein displaying comprises:
- arranging a user-adjustable controller to vertically divide the displayed part of the superimposed model into a first region and a second region, wherein in the first region only a part of the first digital 3D model of the superimposed digital 3D model is rendered, and wherein in the second region only a part of the second digital 3D model of the superimposed digital 3D model is rendered,
- identifying a first end point of the lesion in the first region and a second end point of the lesion in the second region,
- arranging a first interactive window at any point of a display device with an x coordinate less than, or equal to the x coordinate of the first end point of the lesion, the first interactive window comprising a severity value of the lesion identified on the first digital 3D, and
- arranging a second interactive window at any point of the display device with the x coordinate greater than, or equal to
the x coordinate of the second end point of the lesion, the second interactive window comprising a severity value of the lesion identified on the second digital 3D model.

Expression "3D" throughout the present disclosure refers to the term "three-dimensional". Term "digital 3D model" refers to a digital, three-dimensional, computer-generated representation of the patient's dental situation.

Such digital 3D model may accurately correspond to the actual dental situation. That means that dental objects like teeth, teeth surfaces, restorations and/or gingiva on the digital 3D model may correspond to those of the dental situation.

The digital 3D model may be constructed by a processor, based on scan data collected in an intraoral scanning process in which an intraoral 3D scanner may be used to scan the patient's dental situation comprising teeth and gingiva. The intraoral 3D scanner throughout the disclosure is also referred to as the intraoral scanner. The digital 3D model can be stored in a memory of a computer system, for example in a Standard Triangle Language (STL) format or in any other format for displaying or printing 3D objects.

The digital 3D model can be received or accessed by the processor. The digital 3D model may usually be displayed on a display screen in form of a 3D mesh, representing surfaces of teeth and gingival tissue of the dental situation. The 3D mesh may be comprised of individual facets, for example triangular facets while each facet may comprise, for example, three mutually connected vertices. Alternatively, the digital 3D model may be displayed as a point cloud comprising points, a graph comprising nodes and edges, a volumetric representation comprising voxels, or any other suitable 3D representation form.

The method according to an embodiment may comprise receiving the first digital 3D model representative of the dental situation at the first time. The lesion of the dental condition, for example a caries lesion, may be identified on the first digital 3D model.

The method may further comprise receiving the second digital 3D model representative of the dental situation at the second time.

The second time may be later than the first time or may be earlier than the first time. In the exemplary case described herein, the second time is later than the first time. The lesion of the dental condition, for example the previously identified caries lesion, may also be identified on the second digital 3D model. However, the lesion of the dental condition identified on the second digital 3D model may differ from the lesion of the dental condition identified on the first digital 3D model. For example, the severity of the lesion may have changed over a time period between the first and the second time.

The method may additionally comprise generating the superimposed digital 3D model by mutually aligning the first digital 3D model and the second digital 3D model. Aligning the first digital 3D model and the second digital 3D model may comprise globally aligning the first digital 3D model and the second digital 3D model and/or locally aligning the first digital 3D model and the second digital 3D model. As a result of the local alignment, corresponding teeth of the first digital 3D model and the second digital 3D model are more accurately aligned to each other compared to global alignment. Aligning the first digital 3D model and the second digital 3D model may comprise, for example, use of the Iterative Closest Point (ICP) algorithm and/or use of individual coordinate systems of teeth. Corresponding teeth of the first digital 3D model and the second digital 3D model may refer to teeth with the same Universal Numbering System (UNN) dental notation.

A method according to the disclosure may comprise detecting a user-issued command for interaction with one of the first digital 3D model or the second digital 3D model, applying the command to the one of the first digital 3D model or the second digital 3D model and automatically applying a corresponding command to the other one of the first digital 3D model or the second digital 3D model. In this way the first digital 3D model and the second digital 3D model may stay superimposed during the user interaction. The command, for example, may be a command for rotation, translation, pan and/or zoom of a digital 3D model.

The method may further comprise displaying the part of the superimposed digital 3D model comprising the lesion of the dental condition identified on the first digital 3D model and on the second digital 3d model.

Displaying of the part of the superimposed digital 3D model may occur based on user interaction with the superimposed digital 3D model, for example when the user clicks on the lesion of the superimposed digital 3D model, the corresponding part of the superimposed digital 3D model may be zoomed in. While zooming in, the superimposed digital 3D model may be automatically adjusted such that the lesion is in a view direction of the user. This view direction may be referred to as an ideal view direction.

Displaying of the part of the superimposed digital 3D model comprising the lesion of the dental condition may comprise arranging the user-adjustable controller to vertically divide the displayed part of the superimposed model into the first region and the second region, wherein in the first region only the part of the first digital 3D model of the superimposed digital 3D model is rendered, and wherein in the second region only the part of the second digital 3D model of the superimposed digital 3D model is rendered. In this way, a lesion-specific assessment can be made by the user in order to determine the changes in the lesion over time.

The user-adjustable controller may be moved across a graphical user interface, by the user, for example in the left and/or right direction, thereby adjusting the rendering of the first digital 3D model and/or the second digital 3D model of the superimposed digital 3D model. The user may, by adjusting the user-adjustable controller, toggle between rendering the lesion of the dental condition identified on the first digital 3D model and the lesion of the dental condition identified on the second digital 3D model. The user-adjustable controller may also be referred to as a "slider" throughout the disclosure, due to nature of its movement and effect.

The slider may be movable between two end positions. A leftmost position of the slider may result in rendering of only the lesion of the dental condition on the second digital 3D model. A rightmost position of the slider may result in rendering of only the lesion of the dental condition on the first digital 3D model. Any position of the slider in between the two end positions may result in partial rendering of the lesion of both the first digital 3D model and the second digital 3D model.

Displaying of the part of the superimposed digital 3D model comprising the lesion of the dental condition may further comprise identifying the first end point of the lesion in the first region and identifying the second end point of the lesion in the second region. The first end point may be a leftmost point of the lesion of the first digital 3D model visible on the graphical user interface. The second end point may be a rightmost point of the lesion of the second digital 3D model, visible on the graphical user interface.

The leftmost point and the rightmost point may be points on the front-facing facets of the lesion, thereby facing a virtual camera. Any facets of the lesion not facing the virtual camera may be ignored when identifying the first and the second end point. The leftmost point and the rightmost point may additionally or alternatively be determined using only non-occluded control points of the lesion. In this way it may be ensured that the determined first and the second end point are directly visible, by the user, on the graphical user interface, as they are not hidden by any part of the superimposed digital 3D model.

Displaying of the part of the superimposed digital 3D model comprising the lesion of the dental condition may further comprise arranging the first interactive window at any point of the display device with the x coordinate less than, or equal to the x coordinate of the first end point of the lesion. The first interactive window may comprise the severity value of the lesion identified on the first digital 3D model. Further, displaying of the part of the superimposed digital 3D model comprising the lesion may comprise displaying the second interactive window at any point of the display device with the x coordinate greater than, or equal to the x coordinate of the second end point of the lesion. The second interactive window may comprise the severity value of the lesion identified on the second digital 3D model.

The x coordinates of the first and the second end point may refer to values of the x axis of a two-dimensional XY coordinate system of the display device, positioned for example in the midpoint of the display device.

Throughout the disclosure, terms "lesion" and "lesion of the dental condition" are used interchangeably.

In this way, annotations with metadata about the lesion, in the form of the first and second interactive windows, may be positioned with respect to the three-dimensional lesion such as not to impede the view of the lesion.

The first interactive window and the lesion of the first digital 3D model may be mutually arranged such that the first end point is the only point of contact between the lesion of the first digital 3D model and the first interactive window, or such that the first end point is the closest point between the lesion of the first digital 3D model and the first interactive window. Similarly, the second interactive window and the lesion of the second digital 3D model may be mutually arranged such that the second end point is the only point of contact between the lesion of the second digital 3D model and the second interactive window, or such that the second end point is the closest point between the lesion of the second digital 3D model and the second interactive window.

The first interactive window and the second interactive window may comprise the severity value of the corresponding lesion. Additionally, further descriptive information about the corresponding lesion may be comprised in the interactive windows, such as for example type of the lesion, identification number of the lesion, identification number of the affected tooth, size of the lesion in form of surface area covered and/or depth of the lesion.

The first end point may be a leftmost point of the lesion in the first region. Similarly, the second end point may be a rightmost point of the lesion in the second region.

According to the disclosure, the first interactive window may be displayed outside of a border of the lesion of the first digital 3D model. Similarly, the second interactive window may be displayed outside of the border of the lesion of the second digital 3D model. In this way, the interactive windows do not overlap with the displayed lesion and allow the user unobstructed view of the lesion.

The border of the lesion may be represented as a smoothed spline comprising a plurality of control points. In the following, a procedure for identifying the first end point and the second end point based on the plurality of control points of the lesion will be described. In the following, transformation of coordinates of the lesion using transformation matrices, from 3D coordinates into 2D coordinates, will be described.

Each control point of the plurality of control points of the border of the lesion may be transformed from a local 3D space to Normalized Device Coordinates (NDC) of the display device.

This transformation into NDC of the display device may be performed in several steps. Initially, each control point of the plurality of control points of the border of the lesion may be transformed, from the local 3D space, to World space coordinate system using a model matrix (M).

The local 3D space may be a local coordinate system of the lesion. The World space coordinate system may be a global coordinate system of the digital 3D scene. The model matrix may be a transformation matrix that translates, rotates and/or scales the lesion to place it in the World space coordinate system.

Next, each control point of the plurality of control points of the border of the lesion may be transformed from the World space coordinate system to a view space coordinate system using a view matrix (V).

The view space coordinate system may be a coordinate system of a virtual camera representing the user's view. The view matrix (V) may comprise translation and rotation operations to transform the plurality of control points of the border of the lesion to the front of the virtual camera.

Further, each control point of the plurality of control points of the border of the lesion may be transformed from the view space coordinate system to a clip space coordinate system using a projection matrix (P). In the clip space coordinate system a range of coordinates may be defined such that any coordinate outside the defined range is clipped and discarded.

Further, each control point of the plurality of control points of the lesion border may be transformed from the clip space coordinate system to the Normalized Device Coordinates. The Normalized Device Coordinates may range from (0,0,0) to (1,1,1) for a viewport used to represent positions on the display device. Clip space coordinates may be converted to NDC by dividing each component by the homogeneous coordinate.

This sequence of transformations may accurately project a 3D point onto a 2D screen of the display device. The described sequence of transformations from the local 3D space to the Normalized Device Coordinates may be calculated each time the digital 3D scene updates.

The method according to the disclosure may thus comprise updating the transforming of each control point of the plurality of control points of the border of the lesion from the local 3D space to the Normalized Device Coordinates of the display device if a digital 3D scene comprising the displayed part of the superimposed digital 3D model is updated.

The method may thus comprise detecting updating of the digital 3D scene and updating the transforming of each control point of the plurality of control points of the border of the lesion from the local 3D space to the Normalized Device Coordinates of the display device.

Previously described transformation procedure for each control point of the plurality of control points of the border of the lesion may be performed, in an embodiment, only for non-occluded control points of the border of the lesion. Thus, in an embodiment, each control point may be understood as each non-occluded control point. By considering only non-occluded control points, the identification of the first and second end point may be improved.

The digital 3D scene comprising the displayed part of the superimposed digital 3D model may be updated if rotation, translation, panning, zooming in and/or zooming out of the 3D scene is detected. These operations may be done by the user during interaction with the superimposed digital 3D model.

The method may further comprise identifying the first end point as the leftmost point of the lesion in the first region by identifying a point of the border of the lesion in the Normalized Device Coordinates with minimum value of X coordinate.

The method may further comprise identifying the second end point as the rightmost point of the lesion in the second region by identifying a point of the border of the lesion in the Normalized Device Coordinates with maximum value of X coordinate.

The rightmost point and/or the leftmost point may be identified recurringly if the digital 3D scene comprising the displayed part of the superimposed digital 3D model is updated.

The first end point and the second end point, more specifically their x coordinate values, may define the points where the first interactive window and the second interactive window may be placed, respectively. This placement will ensure that all descriptive information about the lesion is available at all times during the user's interaction with the superimposed digital 3D model without covering any area of the lesion itself. Furthermore, the user may edit properties such as the severity of the lesion in the first interactive window and the second interactive window when visually inspecting the lesion and/or comparing the lesion of the first digital 3D model and the lesion of the second digital 3D model.

The lesion may be identified by applying a trained neural network onto the first digital 3D model and/or the second digital 3D model.

Generally, the trained neural network may be trained to detect the lesion of the dental condition directly on a digital 3D model of teeth, or on 2D images of the digital 3D model of teeth.

The trained neural network may be, in an example, a trained convolutional neural network (CNN) configured for detecting the dental condition such as caries, tooth wear, plaque, malocclusion, oral cancer, gingivitis, dental recession or other. A separate trained neural network may be used for detection of different dental conditions. The trained neural network may be adapted to detect presence of the dental condition on a digital 3D model, but also to detect the severity of the dental condition on the digital 3D model.

The trained neural network may be trained on training data comprising annotated digital 3D models on which the dental condition presence and/or severity may be marked by a trained professional.

The user may be able to modify the severity value of the lesion by interacting with the first interactive window and/or the second interactive window. In addition to, or alternatively, the user may be able to modify other parameters of the lesion by interacting with the first interactive window and/or the second interactive window. For example, the user may expand or reduce surface area of the lesion.

The method according to the disclosure may comprise detecting a modified severity value of the lesion in the first interactive window, and re-training the trained neural network based on the first digital 3D model and the modified severity value of the lesion. This advantageously allows the user to improve the behaviour of the trained neural network during its operation.

The method may additionally or alternatively comprise detecting the modified severity value of the lesion in the second interactive window, and re-training the trained neural network based on the second digital 3D model and the modified severity value of the lesion.

The method may therefore comprise transmitting, by the trained neural network, in a feedback loop, the modified severity value and the corresponding digital 3D model to an input of the trained neural network for re-training of the trained neural network.

In an embodiment the re-training of the trained neural network may comprise:
- receiving the first digital 3D model and/or the second digital 3D model and the corresponding modified severity value of the lesion,
- generating an input for re-training of the trained neural network based on the received first digital 3D model and/or the second digital 3D model and the corresponding modified severity value of the lesion,
- providing the input to the trained neural network and obtaining an output comprising a predicted severity value of the lesion,
- comparing the predicted severity value of the lesion to the modified severity value of the lesion to obtain a loss function value,
- updating the weights of the trained neural network based on the loss function value.

The input may comprise an input element of the first and/or second digital 3D model, such as a facet, a point or a vertex, and geometric information associated with the input element.

The geometric information may comprise depth information relating to the virtual camera position, an angle between a facet normal and the virtual camera direction, the facet normal and/or curvature information.

The method according to the disclosure may further comprise detecting a deletion of the lesion in the first interactive window, and re-training the trained neural network based on the first digital 3D model and the deletion of the lesion. This may occur if the user assesses that the lesion was incorrectly detected and decides to delete the lesion by interacting with the first interactive window.

Similarly, the method may comprise detecting the deletion of the lesion in the second interactive window, and re-training the trained neural network based on the second digital 3D model and the deletion of the lesion.

Deletion of the lesion may also occur if the lesion of the dental condition is detected for a dental filling.

Re-training of the trained neural network may comprise, in an embodiment:
- receiving the first digital 3D model and/or the second digital 3D model and information of the deletion of the corresponding lesion,
- generating the input for re-training of the trained neural network based on the received first digital 3D model and/or the second digital 3D model and the information of the deletion of the corresponding lesion,
- providing the input to the trained neural network and obtaining the output comprising a prediction of the lesion presence,
- comparing the prediction of the lesion presence to the information of the deletion of the corresponding lesion to obtain a loss function value,
- updating the weights of the trained neural network based on the loss function value.

Re-training of the neural network according to the disclosure advantageously allows the user to improve the behaviour of the trained neural network, all while directly comparing the lesion of the first digital 3D model to the lesion of the second digital 3D model on the superimposed digital 3D model.

The method may further comprise adjusting a performance metric of the trained neural network based on the detected modified severity value of the lesion. This adjusting of the performance metric may be performed automatically if the modification of the severity value of the lesion is detected. The adjusted performance metric may then be displayed.

The method may comprise adjusting the performance metric of the trained neural network based on detecting of the deletion the lesion. This adjusting of the performance metric may be performed automatically if the deletion of the lesion is detected. The adjusted performance metric may then be displayed.

The method according to the disclosure may comprise detecting a modified parameter of the lesion in the first interactive window and/or the second interactive window, and re-training the trained neural network based on the corresponding digital 3D model and the modified parameter of the lesion. The parameter of the lesion may be surface area of the lesion, volume of the lesion, type of dental condition or other.

In the following, several techniques to track and visualize changes in the lesion will be discussed.

The method may further comprise applying a first color and/or a first transparency value to the first digital 3D model and applying a second color and/or a second transparency value to the second digital 3D model, wherein the first color and/or transparency value is different to the second color and/or second transparency value.

Further, a color gradient may be applied to the lesion to represent changes in the severity value of the lesion or size of the lesion.

Instead of the color gradient, discrete color values may be applied to the lesion to represent changes in the severity value of the lesion or changes in the size of the lesion.

The method may further comprise generating a difference model from the first digital 3D model and the second digital 3D model.

The difference model may be obtained by subtracting the first digital 3D model from the second digital 3D model or by subtracting the second digital 3D model from the first digital 3D model. Then, the first color may be applied to areas of growth in the difference model and the second color may be applied to areas of shrinkage in the difference model.

The difference model may additionally or alternatively be obtained by determining a difference between the surface area of the lesion of the first digital 3D model and the surface area of the lesion of the second digital 3D model. The difference model may also relate to a distance between points on the actual gingiva and points on the ideal gingiva in case the dental condition is recession.

A metric of the lesion may be plotted over time, wherein the time is a time difference between the second time and the first time. The metric of the lesion may be the surface area of the lesion, a lesion depth and/or the severity value of the lesion.

Certain changes of the lesion may be considered normal and within what is expected to occur due to natural passage of time. For example, it may be normal for a certain amount of tooth wear to appear over time, but there may be a threshold of maximum amount of tooth wear expected over a period of time. The method may thus comprise displaying an alert upon determining that the metric of the lesion exceeds a pre-defined threshold.

Additionally, an animation of a transition between the lesion in the first digital 3D model and the lesion in the second digital 3D model may be generated and displayed.

Further, the method may comprise highlighting a change between the lesion in the first digital 3D model and the lesion in the second digital 3D model during displaying of the animation.

Highlighting the change may comprise applying different color and/or texture values to the lesion in the first digital 3D model and the lesion in the second digital 3D model during displaying of the animation.

The dental condition may be caries, dental plaque, tartar, oral cancer, dental recession, tooth wear, gingivitis and/or a tooth crack.

A computer-readable medium is further disclosed, comprising instructions which, when executed by a computer, cause the computer to carry out the method according to the disclosure.

According to the disclosure, a computer program product is further disclosed comprising instructions which, when the program is executed by a computer, causes the computer to carry out any method according to the disclosure.

A dental scanning system comprising a data processing device configured to carry out the method according to the disclosure is further disclosed.

### Brief description of the figures

FIG. 1 schematically illustrates a first digital 3D model of a dental situation and a second digital 3D model of the same dental situation;
FIG. 2 illustrates a part of the superimposed digital 3D model with a lesion of the dental condition divided in two regions by the user-adjustable controller;
FIG. 3 is a flowchart illustrating steps of a method according to the disclosure;
FIG. 4 illustrates steps of identifying a first end point and a second end point of the lesion for placing the interactive windows upon updating of the digital 3D scene;
FIG. 5 illustrates a trained neural network for detecting a dental condition with a feedback loop for re-training of the trained neural network;
FIG. 6 is a flowchart illustrating a training process for a neural network;
FIG. 7 illustrates the part of the superimposed digital 3D model with the lesion of the dental condition divided in two regions having a different severity value in each of the two regions;
FIG. 8 illustrates an intraoral scanning system.

### Detailed description

In the following description, reference is made to the accompanying figures, which show by way of illustration how the invention may be practiced.

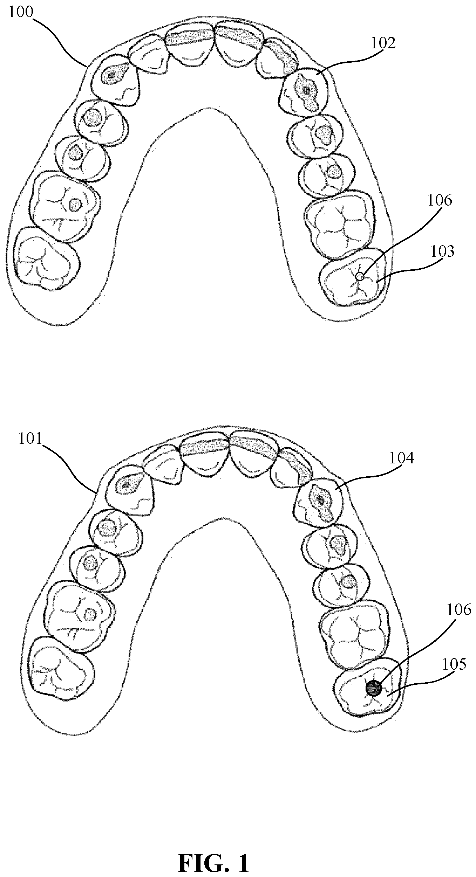

FIG. 1 illustrates a first digital three-dimensional (3D) model 100 representative of a patient's dental situation at a first time and a second three-dimensional (3D) model 101 representative of the same dental situation at a second time. The second time may be later than the first time or earlier than the first time. For exemplary purposes the second time is chosen to be later than the first time. The first digital three-dimensional model 100 may correspond to patient's teeth at a first visit to a dental clinic, while the second digital three-dimensional model 101 may correspond to the patient's teeth at a second visit to a dental clinic.

The first digital 3D model 100 and the second digital 3D model 101 may be different. The differences may be reflected in the appearance and/or progress of one or more dental condition during the time period in between the first time and the second time. Such dental condition may be, for example, tooth wear, illustrated as dark areas on the first 3D model 100 and the second 3D model 101. These dark areas represent a loss in tooth material due to tooth wear. Thus, a difference in geometry between the first and second digital 3D models 100, 101 may exist. The difference in geometry may be a result of change in morphology of teeth due to tooth wear. FIG. 1 illustrates the patient's upper jaw, however the first and/or second digital 3D models 100, 101 may comprise additionally or alternatively the lower jaw or both the upper jaw and the lower jaw.

The first and second digital 3D models 100, 101 may have pairs of corresponding teeth. Corresponding teeth may relate to teeth with the same Universal Numbering System notation (UNN) on the first and second digital 3D model 100, 101. For example, the canine 102 on the first digital 3D model 100 has a corresponding canine 104 on the second digital 3D model 101 with the same UNN notation. Thereby, tooth 102 and its corresponding tooth 104 illustrated in FIG. 1 are an example of corresponding teeth.

It may be observed that an identified lesion of the dental condition on the tooth 102, in this case a tooth wear lesion, has not changed during the time period in between the visits to the dental clinic, as this lesion is the same, in size and in severity, as on the corresponding tooth 104. However, the lesion 106 on the tooth 103 of the first digital 3D model may have changed during the time period in between the visits to the dental clinic, as the global view of FIG. 1 may suggest that the same lesion 106 on the corresponding tooth 105 of the second digital 3D model 101 has increased in size and in severity.

From the user's perspective, an efficient comparison technique is desired to compare, in detail, each of the identified lesions of the dental condition, in order to assess if and how the lesions have changed during time. This is necessary, in order to be able to establish the correct diagnosis and to prescribe a corresponding treatment. Comparing the corresponding lesions on two separately displayed digital 3D models, such as shown in FIG. 1, is not efficient due to the effort needed, by the user, to visually identify corresponding lesions and assess their similarity.

The first digital 3D model 100 and the second digital 3D model 101 may be mutually superimposed. Superimposing and displaying the two digital 3D models may be beneficial as a more efficient tracking of changes between the 3D models may be performed.

If the user selects a particular lesion, for example by clicking on the lesion 106, a part of the superimposed digital 3D model comprising the lesion 106 may be brought in the field of view of the user, thus allowing the user to inspect the selected lesion 106 in more detail.

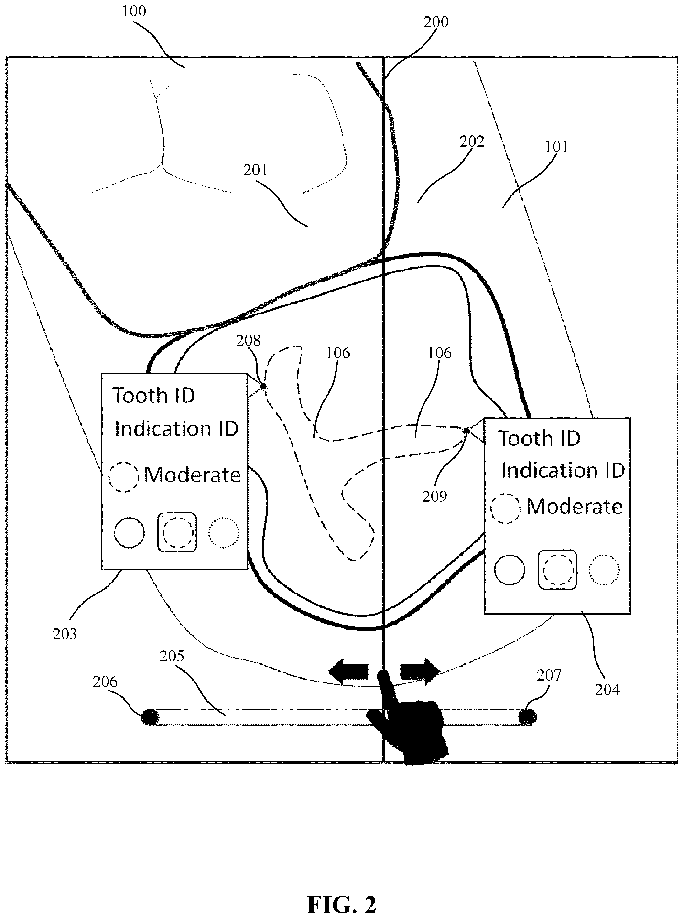

FIG. 2 illustrates displaying the part of the superimposed digital 3D model with the lesion 106 of the dental condition divided in two regions by the user-adjustable controller 200.

The controller 200 may vertically divide the displayed part of the superimposed digital 3D model comprising the lesion 106 into a first region 201 and a second region 202. The controller 200 may also simultaneously vertically divide the lesion 106. The position of the controller 200 with respect to the displayed part of the superimposed digital 3D model may define where a part of the first digital 3D model 100 of the superimposed digital 3D model will be rendered and where a part of the second digital 3D model 101 of the superimposed digital 3D model will be rendered. In the first region 201 only the part of the first digital 3D model 100 of the superimposed digital 3D model may be rendered. In the second region 202 only the part of the second digital 3D model 101 of the superimposed digital 3D model may be rendered.

The first region 201, as shown in FIG. 1, may be a portion of the graphical user interface delimited, on its right side, by the position of the controller 200. Correspondingly, the second region 202 may be a portion of the graphical user interface delimited, on its left side, by the position of the controller 200.

The user may drag the controller 200, for example by using a computer mouse or a touchpad, and thereby observe simultaneously at least a part of the lesion 106 of the first digital 3D model 100 and a part of the lesion 106 of the second digital 3D model 101. In this manner an efficient comparison tool may be provided to the user where any change to the lesion 106 of the dental condition may be observed by simply dragging the controller 200 to the left or to the right of a graphical user interface.

FIG. 2 additionally illustrates a first interactive window 203 and a second interactive window 204. These interactive windows may be understood as 2D annotations associated with the lesion 106. Within these interactive windows at least a severity value of the lesion 106 may be indicated. In addition, information such as tooth identification number and/or identification number of the dental condition may be shown.

FIG. 2 additionally shows a timeline 205 that may indicate time instances when the patient had their dental situation scanned.

For example, a point 206 may correspond to the first time in which the first digital 3D model 100 is representative of the patient's dental situation. A point 207 may correspond to the second time in which the second digital 3D model 101 is representative of the patient's dental situation.

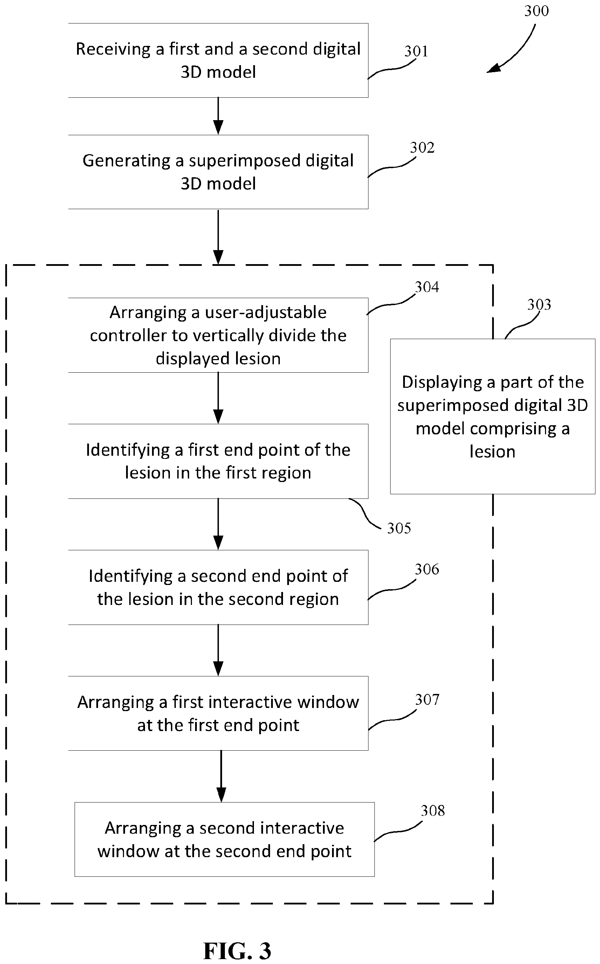

FIG. 3 illustrates a flowchart of a method 300 according to the disclosure.

Step 301 illustrates receiving of the first digital 3D model 100 and the second digital 3D model 101.

In step 302 the superimposed digital 3D model may be generated. The superimposed digital 3D model may be generated by mutually aligning the first digital 3D model 100 and the second digital 3D model 101. Aligning the first digital 3D model 100 and the second digital 3D model 101 may comprise globally aligning the first digital 3D model 100 and the second digital 3D model 101 and/or locally aligning the first digital 3D model 100 and the second digital 3D model 101. As a result of the local alignment, corresponding teeth of the first digital 3D model 100 and the second digital 3D model 101 are more accurately aligned to each other compared to global alignment. Aligning the first digital 3D model 100 and the second digital 3D model 101 may comprise use of the Iterative Closest Point (ICP) algorithm and/or use of individual coordinate systems of teeth. Corresponding teeth of the first digital 3D model 100 and the second digital 3D model 101 may refer to teeth with the same Universal Numbering System (UNN) dental notation.

Step 303 illustrates displaying the part of the superimposed digital 3D model comprising the lesion 106 of the dental condition identified on the first digital 3D model 100 and on the second digital 3d model 101.

Displaying step 303 may comprise arranging the user-adjustable controller 200 to vertically divide the displayed part of the superimposed model into the first region 201 and the second region 202, wherein in the first region 201 only the part of the first digital 3D model 100 of the superimposed digital 3D model is rendered, and wherein in the second region only the part of the second digital 3D model 101 of the superimposed digital 3D model is rendered.

The user-adjustable controller 200 may additionally vertically divide the lesion 106.

Step 305 represents identifying a first end point 208 of the lesion 106 in the first region 201. In the step 306 a second end point 209 of the lesion 106 in the second region 202 may be identified.

Step 307 illustrates arranging a first interactive window 203 at the first end point 208 of the lesion 106, the first interactive window 203 comprising a severity value of the lesion 106 identified on the first digital 3D model 101. The first interactive window 203 may be arranged at any point of a display device with the x coordinate less than, or equal to the x coordinate of the first end point 208.

Step 308 illustrates displaying a second interactive window 204 at the second end point 209 of the lesion 106, the second interactive window 204 comprising the severity value of the lesion 106 identified on the second digital 3D model 101. The second interactive window 204 may be arranged at any point of the display device with the x coordinate greater than, or equal to the x coordinate of the second end point 209.

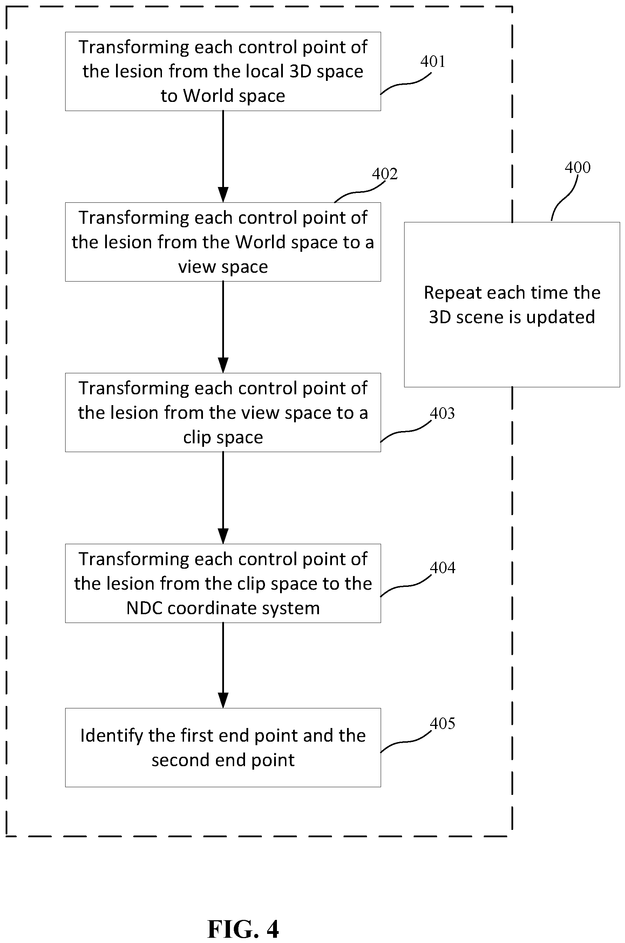

FIG. 4 illustrates steps of identifying the first end point 208 and the second end point 209 of the lesion 106 for placing the interactive windows 203, 204 upon updating of the digital 3D scene. The end points 208, 209 may be dynamically calculated each time the user interacts with the superimposed digital 3D model, thereby updating the digital 3D scene. As effect, this may cause the interactive windows 203, 204 to be constantly positioned at the respective edges of the lesion 106.

Step 400 indicates that the process for identifying the first end point 208 and the second end point 209 may be repeated each time the digital 3D scene is updated.

In step 401 each control point of a plurality of control points of the lesion 106, more specifically of a border of the lesion 106, may be transformed, from the local 3D space, to World space coordinate system using a model matrix (M).

The local 3D space may be a local coordinate system of the lesion 106. The World space coordinate system may be a global coordinate system of the digital 3D scene. The model matrix may (M) be a transformation matrix that translates, rotates and/or scales the lesion 106 to place it in the World space coordinate system.

Transforming each control point of the plurality of control points of the border of the lesion 106, from the World space coordinate system to a view space coordinate system using a view matrix (V) is illustrated in the step 402. The view space coordinate system may be a coordinate system of a virtual camera representing the user's view. The view matrix (V) may comprise translation and rotation operations to transform the plurality of control points of the border of the lesion to the front of the virtual camera.

In the step 403, transforming each control point of the plurality of control points of the border of the lesion 106, from the view space coordinate system to a clip space coordinate system using a projection matrix (P) is illustrated. In the clip space coordinate system a range of coordinates may be defined such that any coordinate outside the defined range is clipped and discarded.

Step 404 illustrates transforming each control point of the plurality of control points of the border of the lesion 106 from the clip space coordinate system to the Normalized Device Coordinates. The Normalized Device Coordinates may range from (0,0,0) to (1,1,1) for a viewport used to represent positions on a virtual plotting device. Clip space coordinates may be converted to NDC by dividing each component by the homogeneous coordinate.

Thereby the steps 401-404 illustrate how each control point of the plurality of control points of the border of the lesion 106 may be transformed from the local 3D space to Normalized Device Coordinates (NDC) of the display device. This sequence of transformations may accurately project a 3D point onto a 2D screen of the display device. The described sequence of transformations from the local 3D space to the Normalized Device Coordinates may be calculated each time the digital 3D scene updates.

Step 405 illustrates identifying the first end point 208 and the second end point 209. The first end point 208 may be identified by locating the leftmost point of the lesion 106 in the first region 201. This may be achieved by identifying a point of the border of the lesion 106 in the Normalized Device Coordinates with minimum value of x coordinate.

The second end point 209 may be identified as the rightmost point of the lesion 106 in the second region 202 by identifying a point of the border of the lesion 106 in the Normalized Device Coordinates with maximum value of x coordinate.

The rightmost point and/or the leftmost point may be identified each time the digital 3D scene comprising the displayed part of the superimposed digital 3D model is updated.

The first end point 208 and the second end point 209 may be used to position the first interactive window 203 and the second interactive window 204, respectively.

The user may interact with the first interactive window 203 and/or the second interactive window 204 and modify the severity value of the lesion 106 upon visually inspecting the lesion 106.

The modified property of the lesion may be used to re-train a trained neural network used to identify the lesion 106 of the dental condition.

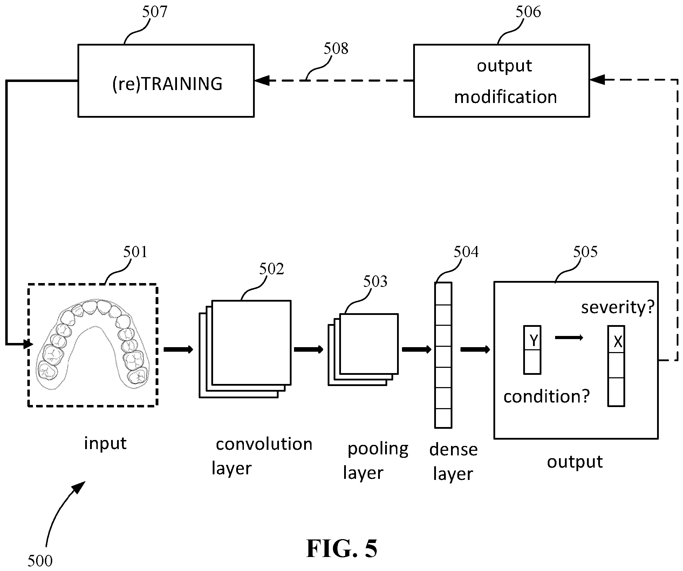

FIG. 5 illustrates a trained neural network 500 for detecting the dental condition with a feedback loop 508 for re-training of the trained neural network 500.

The trained neural network 500 may be a convolutional neural network and may comprise a plurality of convolution layers 502 capable of capturing low-level features of an input 501, i.e. obtaining convolved features. Moreover, one or more of pooling layers 503 may be present, responsible for reducing the spatial size of convolved features. Convolutional neural networks may be particularly suitable for processing matrix information such as 2D images.

The input 501 may be in a 2D or 3D format and may comprise at least one input element such as a tooth, a facet of a tooth, a point of a tooth point cloud and/or a voxel. Figure 5 illustrates the input as being a digital 3D model of a jaw. The at least one input element may be a 2D image of a tooth of the jaw which has one of its surfaces affected by the dental condition. The at least one input element may alternatively comprise one or more of 2D images of a surface the tooth or at least one pixel of the 2D image of the tooth.

The output 505 may comprise the at least one probability value representing the likelihood that the dental condition is present in the input 501 and/or may classify the dental condition further into a severity category. For example, the lesion of caries may be detected in the input 501 and a moderate severity value may be assigned to it. Severity values may also be mild or severe, for example.

Further shown in FIG. 5 is the block 506 illustrating detecting of the output 505 modification by the user. The user may compare the lesion 106 of the first digital 3D model 100 to the lesion 106 of the second digital 3D model 101, for example in a tool presented on FIG. 2. The user may interact with the first interactive window 203 and/or the second interactive window 204 and modify the severity value of the lesion 106. For example, the user may change the severity value of the lesion 106 in the second interactive window from "moderate" to "severe".

Once the output 505 modification is detected, a feedback loop 508 may be introduced to re-train the trained neural network 500 based on the output 505 modification. The trained neural network 500 may transmit, in the feedback loop 508, the modified severity value and the corresponding digital 3D model to the input 501 of the trained neural network 500 for re-training of the trained neural network 500.

In the process of re-training of the trained neural network 500 the modified severity value may be used as a ground truth and weights of the trained neural network 500 may be adjusted by comparing the output of the trained neural network to this ground truth.

The output modification 506 may comprise detecting a deletion of the lesion 106 in the first interactive window 203 and/or in the second interactive window 204. The user may decide to delete the lesion 106 for example if the trained neural network 500 has indicated lesion 106 presence on a dental filling. This information may also be used to re-train the trained neural network 500 through the feedback loop 508.

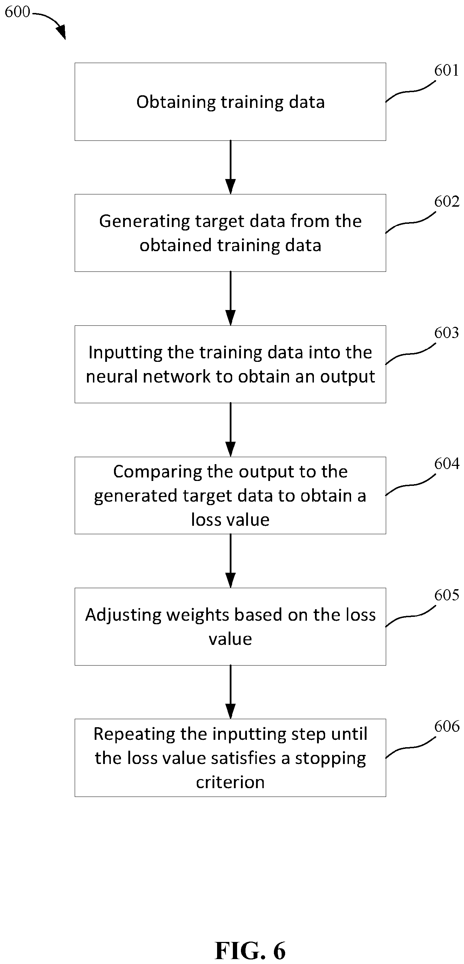

FIG. 6 illustrates a flowchart of a training process for a neural network for detecting the lesion 106 of the dental condition.

The neural network may be trained for a classification task. A training process for the classification task of the trained neural network 500 may be described as follows. The overall aim of a non-trained neural network may be to develop the trained neural network 500 capable of classifying the input 501 according to presence of the lesion of the dental condition and/or the severity value of the lesion.

The training process may be referred to as "supervised training" due to use of a training data that has been labeled. Step 601 illustrates obtaining of the training data. The training data may comprise at least 1000 three-dimensional digital models which can yield over 17000 individual teeth. The training data may be in 2D format, i.e. it may comprise a plurality of 2D training images of teeth or parts of teeth. The training data may alternatively be in 3D format comprising a plurality of teeth represented as 3D meshes, 3D graphs or point clouds.

Step 602 illustrates generating target data from the obtained training data. This may refer to annotating the training data to obtain the ground truth data. The target data may thus comprise labels indicating presence of the dental condition on the training data. The annotating, also referred to as labeling, may be performed for example by a qualified dental practitioner. The training data may be labeled according to the dental condition presence, for example with "Yes" or "No" labels indicating the presence or absence of the dental condition. Additionally or alternatively, the training data may be labeled with the severity value labels associated with corresponding lesions.

The labels may be regarded as the target or the ground truth for a given training data set.

The training data may be fed into the non-trained neural network, illustrated in the step 603. Output of the non-trained neural network may be obtained, comprising a probability value indicating which output class the input, or an input element, belongs to.

A loss may be determined by comparing the output to the generated target data, illustrated in the step 604. This loss may be determined by a loss function. One example of the loss function may be a mean-squared-error function. In order to train the non-trained neural network, this loss function should be minimized. The minimization of the loss function may be achieved for example by using a stochastic gradient descent algorithm.

Through this process of minimizing the loss function, weights of the non-trained neural network may be updated, illustrated in the step 605.

The training process may be an iterative process in which the same training data set may be fed, iteratively, into the non-trained neural network until a stopping criterion is reached, illustrated in the step 606. The stopping criterion may be a specific threshold value for the loss function. The stopping criterion may be a number of epochs (training cycles) after which performance metrics cease to increase.

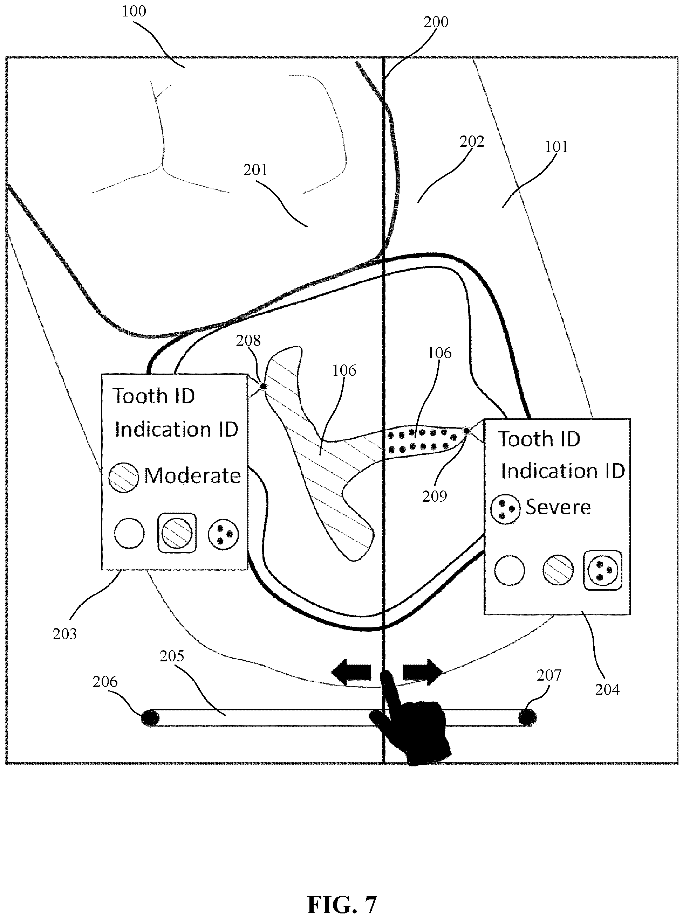

FIG. 7 illustrates the part of the superimposed digital 3D model with the lesion 106 of the dental condition divided in two regions 203, 204 having a different severity value in each of the two regions 203, 204. FIG. 7 illustrates same elements as

FIG. 2, with the difference in how the lesion 106 is generated.

Firstly, in FIG. 7 it can be seen that the lesion 106 of the dental condition in the second region 202, thereby in the second digital 3D model 101 of the superimposed digital 3D model, is marked as having the severity level as "severe", while the lesion 106 in the first region 201 has the severity level "moderate". These severity levels may be assigned by the trained neural network 500 or adjusted manually by the user by interacting with the first interactive window 203 and/or the second interactive window 204.

Furthermore, FIG. 7 illustrates how the lesion 106, together with the inside surface defined by the lesion border may be presented in order to provide an efficient comparison tool.

A first color and/or a first transparency value may be applied to the first digital 3D model 100, or to the lesion 106 of the first digital 3D model 100, while a second color and/or a second transparency value may be applied to the second digital 3D model 101, or to the lesion 106 of the second digital 3D model 101. The first color and/or transparency value may be different to the second color and/or second transparency value.

Alternatively or additionally, a color gradient or discrete color values may be applied to the lesion 106 to represent changes in the severity value of the lesion 106 or in size of the lesion 106.

Further features, not shown in FIG. 7 may be introduced to improve the efficiency of the comparison tool. For example, a difference model may be generated from the first digital 3D model 100 and the second digital 3D model 101 by subtracting the first digital 3D model 100 from the second digital 3D model 101 or by subtracting the second digital 3D model 101 from the first digital 3D model 100. The first color may then be applied to areas of growth in the difference model and/or the second color may be applied to areas of shrinkage in the difference model. Furthermore, a metric of the lesion 106 over time may be plotted, wherein the time is a time difference between the second time and the first time. The metric of the lesion 106 may be a surface area or the severity value of the lesion 106. Additionally or alternatively an animation of a transition between the lesion 106 in the first digital 3D model 100 and the lesion 106 in the second digital 3D model 101 may be generated and displayed. Thereby a change between the lesion 106 in the first digital 3D model 100 and the lesion 106 in the second digital 3D model 101 may be highlighted. Highlighting of the change may alternatively or additionally comprise applying different color and/or texture values to the lesion 106 in the first digital 3D model 100 and to the lesion 106 in the second digital 3D model 101 during displaying of the animation.

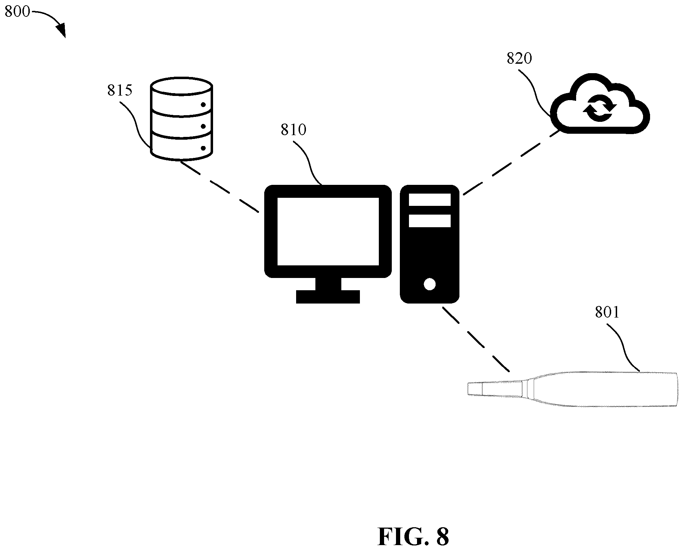

FIG. 8 illustrates a dental scanning system 800 which may comprise a computer 810 capable of carrying out any method of the disclosure. The computer may comprise a wired or a wireless interface to a server 815, a cloud server 820 and the intraoral scanner 801. The intraoral scanner 801 may be capable of recording the scan data comprising geometrical information, natural color information and/or fluorescence information associated with the patient's dentition. The intraoral scanner 801 may be equipped with various modules such as a fluorescence module or an infrared module and thereby capable of capturing information relevant for diagnosing dental conditions such as caries, dental recession, tooth cracks, gingivitis and/or plaque.

The dental scanning system 800 may comprise a data processing device configured to carry out the method according to one or more embodiments of the disclosure. The data processing device may be a part of the computer 810, the server 815 or the cloud server 820.

A non-transitory computer-readable storage medium or a computer-readable storage medium may be comprised in the dental scanning system 800. The non-transitory computer-readable medium can carry instructions which, when executed by a computer, cause the computer to carry out the method according to one or more embodiments of the disclosure.

The computer-readable storage medium may comprise an optical storage media such as an optical disc, a machine-readable bar code, a USB flash memory, a magnetic storage device, a solid-state electronic storage devices such as random access memory (RAM), or read only memory (ROM), or any other physical device or medium employed to store a computer program. Thereby the embodiments of the disclosure can be utilized on a data processing hardware apparatus, such as a computer system or personal computer, or on an embedded system that employs a dedicated data processing unit, such as a digital signal processing chip.

A computer program product may be embodied in the non-transitory computer-readable storage medium. The computer program product may comprise instructions which, when executed by a computer, cause the computer to perform the method according to any of the embodiments presented herein.

References throughout this disclosure to certain features, advantages, or similar does not imply that all of the features and advantages of the present disclosure should be or are in any single embodiment. Instead, it is to be understood that a specific feature, or characteristic described in connection with an embodiment is included in at least one embodiment of the disclosure. Thus, discussion of the features and advantages, and similar language, throughout this disclosure may, but do not necessarily, refer to the same embodiment.

## Claims

1. A computer-implemented method for evaluating digital 3D models of teeth, the method comprising:
- receiving a first digital 3D model representative of the dental situation at a first time;
- receiving a second digital 3D model representative of the dental situation at a second time;
- generating a superimposed digital 3D model by mutually aligning the first digital 3D model and the second digital 3D model;
- displaying a part of the superimposed digital 3D model comprising a lesion of a dental condition identified on the first digital 3D model and on the second digital 3d model, wherein displaying comprises:
- arranging a user-adjustable controller to vertically divide the displayed part of the superimposed model into a first region and a second region, wherein in the first region only a part of the first digital 3D model of the superimposed digital 3D model is rendered, and wherein in the second region only a part of the second digital 3D model of the superimposed digital 3D model is rendered;
- identifying a first end point of the lesion in the first region and a second end point of the lesion in the second region;
- arranging a first interactive window at any point of a display device with an x coordinate less than, or equal to the x coordinate of the first end point of the lesion, the first interactive window comprising a severity value of the lesion identified on the first digital 3D model; and
- arranging a second interactive window at any point of the display device with the x coordinate greater than, or equal to the x coordinate of the second end point of the lesion, the second interactive window comprising a severity value of the lesion identified on the second digital 3D model.

2. The method according to the previous claim 1, wherein the first end point is a leftmost point of the lesion in the first region, and the second end point is a rightmost point of the lesion in the second region.

3. The method according to any previous claim, wherein a border of the lesion is represented as a smoothed spline comprising a plurality of control points.

4. The method according to the previous claim 3, wherein the leftmost point of the lesion and the rightmost point of the lesion are determined considering only non-occluded control points of the smoothed spline and/or front-facing facets of the lesion.

5. The method according to the previous claim 3 or 4, further comprising transforming each control point of the plurality of control points of the border of the lesion from a local 3D space to Normalized Device Coordinates (NDC) of a display device.

6. The method according to the previous claim 5, further comprising updating the transforming of each control point of the plurality of control points of the border of the lesion from the local 3D space to the Normalized Device Coordinates of the display device if a digital 3D scene comprising the displayed part of the superimposed digital 3D model is updated.

7. The method according to the previous claim 5 or 6, further comprising identifying the first end point as the leftmost point of the lesion in the first region by identifying a point of the border of the lesion in the Normalized Device Coordinates with a minimum value of x coordinate.

8. The method according to any previous claim 5-7, further comprising identifying the second end point as the rightmost point of the lesion in the second region by identifying a point of the border of the lesion in the Normalized Device Coordinates with a maximum value of x coordinate.

9. The method according to any previous claim, wherein the first interactive window is displayed outside of the border of the lesion of the first digital 3D model.

10. The method according to any previous claim, wherein the second interactive window is displayed outside of the border of the lesion of the second digital 3D model.

11. The method according to any previous claim, wherein the lesion is identified by applying a trained neural network onto the first digital 3D model.

12. The method according to the previous claim 11, further comprising detecting a modified severity value of the lesion in the first interactive window, and re-training the trained neural network based on the first digital 3D model and the modified severity value of the lesion.

13. The method according to any previous claim, further comprising generating a difference model from the first digital 3D model and the second digital 3D model by subtracting the first digital 3D model from the second digital 3D model or by subtracting the second digital 3D model from the first digital 3D model.

14. The method according to the previous claim 13, further comprising applying a first color to areas of growth in the difference model and applying a second color to areas of shrinkage in the difference model.

15. The method according to any previous claim, further comprising applying a color gradient to the lesion to represent changes in the severity value of the lesion or size of the lesion.
